# DEMANDE DE BREVET EUROPEEN

(11) **EP 4 450 049 A1**
(43) Date de publication de la demande: **23.10.2024**
(21) Numéro de dépôt: 23305591.2
(22) Date de dépôt: 18.04.2023
(51) Int. Cl.: A61K 8/02, A61K 8/29, A61K 8/365, A61K 8/44, A61K 8/73, A61K 8/9728, A61Q 1/02, A61Q 19/00

(54) **COMPOSITION À BASE DE CHITOSAN ET DE PIGMENT AYANT SUBI UN TRAITEMENT DE SURFACE SPÉCIFIQUE**

(71) Demandeur: L'OREAL, 75008 Paris (FR)
(72) Inventeur: LE VERGE, Danielle, 94550 CHEVILLY LARUE (FR); KUSINA, Christophe, 94550 CHEVILLY LARUE (FR)
(74) Mandataire: Lavoix

(57) **Abrégé**

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment traité en surface par un agent choisi parmi les N-acyl amino acides et leurs sels.

Elle se rapporte également à un procédé de maquillage et/ou de soin de la peau et/ou des phanères, dans lequel on applique la composition selon l'invention sur la peau et/ou les phanères.

## Description

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment traité en surface par un agent choisi parmi les N-acyl amino acides et leurs sels.

Elle se rapporte également à un procédé de maquillage et/ou de soin de la peau et/ou des phanères, dans lequel on applique la composition selon l'invention sur la peau et/ou les phanères.

Il existe de nombreuses compositions cosmétiques pour lesquelles des propriétés de tenue du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des agents filmogènes. Cependant, la présence de tels agents pour la tenue du film sur les matières kératiniques peut conduire à des compositions desséchantes.

Par ailleurs, on cherche souvent à obtenir des compositions couvrantes.

Le formulateur est donc à la recherche de matières premières et/ou de systèmes permettant d'obtenir des compositions colorées fluides (i.e. qui s'écoulent) dont le dépôt est couvrant, homogène et résistant (i.e. présentant une bonne tenue), en particulier présentant une bonne tenue à l'eau.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a pour but de proposer des compositions cosmétiques colorées aqueuses présentant de bonnes propriétés maquillantes, notamment en termes de couvrance et d'homogénéité, mais aussi ayant une bonne tenue, en particulier ayant une bonne tenue/résistance à l'eau.

Après application, ces compositions laissent un dépôt filmogène couvrant et homogène, qui a une bonne tenue à l'usure. Les films colorés formés sont adhésifs et cohésifs, et présentent une résistance améliorée à l'eau.

Ces compositions comprennent également des ingrédients durables, permettant ainsi de répondre aux enjeux environnementaux.

La présente invention a donc pour objet une composition cosmétique, notamment de maquillage et/ou de soin de la peau et/ou des lèvres, en particulier des lèvres, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment traité en surface par un agent choisi parmi les N-acyl amino acides et leurs sels.

Par « physiologiquement acceptable », on entend un milieu compatible avec les matières kératiniques.

Elle a également pour objet un procédé de maquillage et/ou de soin de la peau et/ou des phanères, dans lequel on applique la composition selon l'invention sur la peau et/ou les phanères.

### Chitosan

La composition selon l'invention comprend au moins 0,01% en poids par rapport au poids total de la composition d'au moins un chitosan natif ayant un poids moléculaire strictement supérieur à 3000 Daltons (3 kDa).

La quantité de chitosan natif est également strictement inférieure à 15% en poids par rapport au poids total de la composition.

De préférence, le chitosan natif a un poids moléculaire supérieur ou égal à 10 kDa, de préférence supérieur ou égal à 15 kDa, de préférence supérieur ou égal à 20 kDa. De préférence, le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine)-poly(D-glucosamine).

De préférence, le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.*

Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

### Pigment traité en surface

La composition selon l'invention comprend au moins un pigment traité en surface par un agent choisi parmi les N-acyl amino acides et leurs sels.

On entend par « pigments » des particules blanches ou colorées, minérales ou organiques, insolubles dans un milieu aqueux, destinées à colorer la composition et/ou le dépôt résultant.

Les pigments traités peuvent être présents, dans la composition, en une teneur allant de 1% à 70% en poids, de préférence de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids.

### Pigments minéraux

Selon un mode de réalisation particulier, les pigments utilisés selon l'invention sont choisis parmi les pigments minéraux.

Par « pigment minéral », on entend tout pigment qui répond à la définition de l'encyclopédie Ullmann dans le chapitre pigment inorganique. On peut citer, parmi les pigments minéraux utiles dans la présente invention, le dioxyde de titane, les oxydes de fer (noir, jaune ou rouge), les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre. Les pigments minéraux suivants peuvent aussi être utilisés : Ta2O5, Ti3O5, Ti2O3, TiO, ZrO2 en mélange avec TiO2, ZrO2, Nb2O5, CeO2, ZnS.

La taille du pigment utile dans le cadre de la présente invention est en général supérieure à 100 nm et peut aller jusqu'à 10 µm, de préférence de 200 nm à 5 µm, et plus préférentiellement de 300 nm à 1 µm. Selon une forme particulière de l'invention, les pigments présentent une taille caractérisée par un D[50] supérieur à 100 nm et pouvant aller jusqu'à 10 µm, de préférence de 200 nm à 5µm, et plus préférentiellement de 300 nm à 1 µm. Les tailles sont mesurées par diffusion statique de la lumière au moyen d'un granulomètre commercial de type MasterSizer 3000^{®} de chez Malvern, permettant d'appréhender la répartition granulométrique de l'ensemble des particules sur une large gamme pouvant aller de 0,01 µm à 1000 µm. Les données sont traitées sur la base de la théorie classique de diffusion de Mie. Cette théorie est la plus adaptée pour des distributions de taille allant du submicronique au multi-micronique, elle permet de déterminer un diamètre « effectif » de particules. Cette théorie est notamment décrite dans l'ouvrage de Van de Hulst, H.C., « Light Scattering by Small Particles », Chapitres 9 et 10, Wiley, New York, 1957. D[50] représente la taille maximale que présente 50 % en volume les particules.

Dans le cadre de la présente invention, les pigments minéraux sont plus particulièrement l'oxyde de fer et/ou le dioxyde de titane.

Comme pigments minéraux utilisables dans l'invention, on peut également citer les nacres. Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques. On peut également citer, à titre d'exemple de nacres, le mica naturel recouvert d'oxyde de titane, d'oxyde de fer, de pigment naturel ou d'oxychlorure de bismuth. Les nacres peuvent plus particulièrement posséder une couleur ou un reflet jaune, rose, rouge, bronze, orangé, brun, or et/ou cuivré.

Parmi les pigments utilisables selon l'invention, on peut également citer ceux à effet optique différent d'un simple effet de teinte conventionnel, c'est-à-dire unifié et stabilisé tel que produit par les matières colorantes classiques, comme par exemple, les pigments monochromatiques.

Au sens de l'invention, « stabilisé » signifie dénué d'effet de variabilité de la couleur avec l'angle d'observation ou encore en réponse à un changement de température. Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

### Pigments organiques

Selon un autre mode de réalisation de l'invention, la matière colorante pigmentaire est un pigment organique, synthétique, naturel ou d'origine naturelle.

Par « pigment organique », on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

Le ou les pigments organiques peuvent être choisis par exemple parmi le carmin, le noir de carbone, le noir d'aniline, la mélanine, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, et les pigments obtenus par polymérisation oxydante de dérivés indoliques, phénoliques tels qu'ils sont décrits dans le brevet FR 2 679 771.

Les pigments peuvent aussi être sous forme de pigments composites tels qu'ils sont décrits dans le brevet EP 1 184 426. Ces pigments composites peuvent être composés notamment de particules comportant un noyau inorganique recouvert au moins partiellement d'un pigment organique et au moins un liant assurant la fixation des pigments organiques sur le noyau.

Le pigment peut aussi être une laque.

Par laque, on entend les colorants insolubilisés adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants organiques, on peut citer le carmin de cochenille. On peut également citer les produits connus sous les dénominations suivantes : D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 1 O (CI 77 002), D&C Green 3 (CI 42 053), D&C Blue 1 (CI 42 090). A titre d'exemples de laques, on peut citer le produit connu sous la dénomination D&C Red 7 (CI 15 850 :1).

De préférence, le pigment de l'invention est choisi parmi les pigments minéraux, de préférence parmi le dioxyde de titane, les oxydes de fer, les oxydes de zirconium ou de cérium, les oxydes de zinc ou de chrome et leurs mélanges. De préférence, le pigment de l'invention est choisi parmi le dioxyde de titane, les oxydes de fer et leurs mélanges.

### Agent de traitement de surface

L'agent de traitement de surface selon l'invention est un agent hydrophobe, il est choisi parmi les N-acyl amino acides et leurs sels.

Le N-acyl amino acide est un acide aminé présentant un groupement acyle sur l'azote de l'acide aminé.

L'acide aminé peut être, par exemple, la lysine, l'acide glutamique ou l'alanine. De préférence, l'acide aminé est l'acide glutamique.

Le N-acyl amino acide peut être sous forme d'acide libre ou sous forme partiellement ou totalement salifiée. Lorsqu'il est sous forme salifiée, il s'agit notamment de sels alcalins tels que les sels de sodium, de potassium ou de lithium, de sels alcalino-terreux tels que les sels de calcium, de magnésium ou de strontium ; de sel d'aluminium, de zirconium ou de zinc ; de sel d'ammonium ou de sel d'un amino-alcool comme le sel de (2-hydroxy éthyl) ammonium. De préférence, les sels sont des sels d'aluminium, de magnésium, de calcium, de zirconium, de zinc, de sodium ou de potassium.

Le N-acyl amino acide peut comprendre un groupe acyle ayant de 8 à 22 atomes de carbone, tel que, par exemple, un groupe 2-éthylhexanoyle, caproyle, lauroyle, myristoyle, palmitoyle, stéaroyle ou cocoyle. De préférence, le groupe acyle est un groupe stéaroyle.

De préférence, le N-acyl amino acide est un dérivé d'acide glutamique et/ou un de ses sels, et plus particulièrement un stéaroyl glutamate, par exemple le stéaroyl glutamate de disodium ou le stéaroyl glutamate d'aluminium.

Il s'agit par exemple du traitement de surface NAI (stéaroyl glutamate de disodium) vendu par Miyoshi.

De préférence, le pigment ayant subi un traitement de surface selon l'invention est choisi parmi :
- le dioxyde de titane anatase enrobé de stéaroyl glutamate d'aluminium (97/3) (CI : 77891) (nom INCI : titanium dioxide (and) disodium stearoyl glutamate (and) aluminium hydroxide ; NAI-TAO-77891 ou NAI-White A de MIYOSHI KASEI) ;
- les oxydes de fer traités par le couple stéaroyl glutamate de disodium/hydroxyde d'aluminium, vendus notamment sous la dénomination commerciale NAI-BHP-10, NAI-YHP-10 et NAI-R-800HP-10 par la société Miyoshi Kasei ; et
- les oxydes de fer jaune, rouge ou noir traités par le stéaroyl glutamate d'aluminium (références NAI-C33-9001-10 NAI-C33-8001-10, NAI-C33-7001-10 de MIYOSHI KASEI).

### Milieu aqueux physiologiquement acceptable

La composition selon l'invention comprend un milieu aqueux physiologiquement acceptable. Ledit milieu comprend de l'eau.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle.

La composition comprend de préférence au moins 5% en poids d'eau par rapport au poids total de la composition, de préférence au moins 10% en poids, de préférence au moins 20% en poids, de préférence au moins 30% en poids, de préférence au moins 40% en poids. La composition comprend de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50%.

La phase aqueuse peut également comprendre au moins un solvant organique miscible dans l'eau à 25°C.

De préférence, le solvant organique miscible dans l'eau est choisi parmi les alcools, les polyols et leurs mélanges.

Parmi les alcools, on peut citer les alcools en C₁-C₁₀, plus préférentiellement en C₁-C₅, tels que l'éthanol, l'isopropanol, le propanol et le butanol.

Le polyol est, de préférence, choisi parmi les polyols ayant de 2 à 20 atomes de carbone, plus préférentiellement de 2 à 6 atomes de carbone, comme le glycérol, le diglycérol, le propylèneglycol, l'isoprène glycol, le dipropylèneglycol, le butylène glycol, l'hexylène glycol, le 1,2-propanediol, le 1,3-propanediol, le pentylène glycol, les polyéthylèneglycols ayant de 2 à 200 motifs d'oxyde d'éthylène et leurs mélanges.

La composition peut comprendre de 1% à 25% en poids de solvant organique miscible dans l'eau, par rapport au poids total de la composition, plus préférentiellement de 2% à 20% en poids, encore plus préférentiellement de 3% à 15% en poids.

### Alpha hydroxy acide (AHA)

La composition selon l'invention peut comprendre au moins un AHA.

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.

Les α-hydroxyacides (alpha hydroxyacides ou AHA) sont par exemple choisis parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

Selon un mode préféré, l'alpha hydroxyacide est choisi parmi l'acide lactique, l'acide citrique, l'acide malique, l'acide tartrique et leurs sels. Plus particulièrement, l'alpha hydroxyacide est choisi parmi l'acide lactique, l'acide citrique, leurs sels et leurs mélanges.

Le ou les alpha hydroxyacides peuvent être présents en une quantité allant de 0,001 à 10% en poids, de 0,005 à 5% en poids, de préférence de 0,01 à 3% en poids par rapport au poids total de la composition.

### pH de la composition

La composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

De préférence, la composition cosmétique selon l'invention comprend au moins une base et/ou au moins un acide. La base et l'acide selon l'invention sont connus et classiquement utilisés dans le domaine cosmétique.

La base et/ou l'acide selon l'invention sont notamment utilisés pour ajuster le pH final de la composition entre 3 et 6,3.

L'acide peut par exemple être l'acide citrique.

La base peut être choisie parmi les bases minérales comme par exemple les hydroxydes de métaux alcalins, l'hydroxyde de sodium, l'hydroxyde de potassium.

De préférence, la base de la composition est un hydroxyde de métal alcalin, de préférence l'hydroxyde de sodium ou l'hydroxyde de potassium.

La composition selon l'invention peut comprendre au moins une base en une teneur en matière active allant de 0,5 % à 10 % en poids, par rapport au poids total de la composition, notamment de 1 % à 5 % en poids, de préférence allant de 1 % à 4 % en poids.

La composition conforme à l'invention peut être obtenue de manière classique par l'homme du métier.

Elle a également pour objet un procédé de maquillage et/ou de soin de la peau et/ou des phanères, dans lequel on applique la composition selon l'invention sur la peau et/ou les phanères.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières par rapport au poids total de composition (% p/p), sauf mention contraire.

### Exemples : préparation d'une composition selon l'invention et comparaison avec des compositions comparatives

Les compositions B selon l'invention, et C et H comparatives, ont été préparées par mélange des ingrédients du tableau 1.

La composition B selon l'invention contient des pigments traités en surface par le stéaroyl glutamate comme N-acyl amino acide.

La composition comparative C contient des pigments non traités en surface (natifs).

La composition comparative H contient des pigments traités en surface par la dilauramidoglutamide lysine de sodium (qui n'est pas un N-acyl amino acide).

La composition B selon l'invention présente une meilleure empreinte carbone notamment en favorisant l'emploi de matières premières renouvelables et/ou avec un bon index de naturalité et/ou d'origine naturelle et plus particulièrement d'origine végétale.

Puis la tenue à l'eau est évaluée. Le protocole d'évaluation est le suivant :
- Appliquer chaque composition sur une zone de chaque avant-bras (avec une zone d'avant-bras supplémentaire = témoin) : pour cela, on dépose 10µl de chaque composition sur une zone d'avant-bras de surface 2,5 x 2,5 cm pendant 15 s,
- Laisser sécher 20 min,
- Déposer 100 µL d'eau sur un tissu de frottement en coton,
- Appliquer le coton sur la zone traitée à l'aide d'un poids de 900 g pendant 5 s,
- Retirer le coton avec une force comprise entre 100 et 300 g, et
- Evaluer visuellement le dépôt témoin versus le dépôt frotté à l'eau. Cette évaluation comprend un scorage de tenue à l'eau, qui va de (-) = aucune tenue à l'eau, à (+++++) = excellente tenue à l'eau.

Les résultats sont dans le tableau 1.

**[Table 1]**

| **Ingrédients (% p/p)** | **B selon l'invention** | **C comparative** | **H comparative** |
|---|---|---|---|
| Chitosan | 2 | 2 | 2 |
| Acide lactique | 1 | 1 | 1 |
| Mélange de pigments de dioxyde de titane (TiO2) et d'oxydes de fer non traités en surface (Tarox Iron Oxide R-800HP, BL-100HPL et LL-100HP de Titan Kogyo et Hombitan FF Pharma de Venator) | - | 15 | - |
| Mélange de pigments de TiO2 traité en surface par du stéaroyl glutamate et d'oxydes de fer traités par le couple stéaroyl glutamate de | 15 | - | - |
| disodium/hydroxyde d'aluminium (NAI-White A et NAI-BHP-10, NAI-YHP-10 et NAI-R-800HP-10 de Miyoshi Kasei) | | | |
| Mélange de pigments de TiO2 et d'oxydes de fer traités en surface par la dilauramidoglutamide lysine de sodium (ASL-1 TIO2 CR-50, ASL-1 yellow LL-100P, ASL-2 red R-516P et ASL-1 black BL-100P de Daito Kasei Kogyo) | - | - | 15 |
| Eau | Qsp 100 | Qsp 100 | Qsp 100 |
| **Note tenue à l'eau (de (-) à (+++++))** | 3 | 1 | 1 |

Les résultats montrent une meilleure tenue à l'eau pour la composition B selon l'invention, par rapport aux compositions comparatives C et H. Le pigment traité en surface par du disodium stéaroyl glutamate, selon l'invention, apporte ainsi une meilleure résistance à l'eau.

## Revendications

1. Composition cosmétique, notamment de maquillage et/ou de soin de la peau et/ou des lèvres, en particulier des lèvres, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment traité en surface par un agent choisi parmi les N-acyl amino acides et leurs sels.

2. Composition selon la revendication 1, dans laquelle le chitosan natif a un poids moléculaire supérieur ou égal à 10 kDa, de préférence supérieur ou égal à 15 kDa, de préférence supérieur ou égal à 20 kDa, de préférence le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

3. Composition selon la revendication 1 ou 2, dans laquelle le chitosan natif a un degré d'acétylation du chitosan inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

4. Composition selon l'une des revendications précédentes, dans laquelle le chitosan natif est un polysaccharide préparé à partir d'une origine fongique, de préférence issu du mycélium d'un champignon du type Ascomycète, et en particulier d'*Aspergillus niger* et/ou d'un champignon Basidiomycète, et en particulier *Lentinula edodes* et/ou *Agaricus bisporus*, de préférence le champignon est *Aspergillus niger.*

5. Composition selon l'une des revendications précédentes, dans laquelle le chitosan natif est présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

6. Composition selon l'une des revendications précédentes, qui comprend de 1% à 70% en poids, de préférence de de 1% à 60% en poids, de préférence de 1% à 50% en poids, de préférence de 3% à 45% en poids, par rapport au poids de la composition, de préférence de 4% à 30% en poids, de préférence de 5% à 20% en poids, de préférence de 6% à 15% en poids, de pigment(s) traité(s).

7. Composition selon l'une des revendications précédentes, dans laquelle le pigment est un pigment minéral choisi parmi le dioxyde de titane, les oxydes de fer, les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre, les nacres, les pigments monochromatiques.

8. Composition selon l'une des revendications précédentes, dans laquelle le pigment est un pigment organique choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

9. Composition selon l'une des revendications précédentes, dans laquelle le pigment est choisi parmi les pigments minéraux, de préférence parmi le dioxyde de titane, les oxydes de fer, les oxydes de zirconium ou de cérium, les oxydes de zinc ou de chrome et leurs mélanges, de préférence le pigment est choisi parmi le dioxyde de titane, les oxydes de fer et leurs mélanges.

10. Composition selon l'une des revendications précédentes, dans laquelle l'acide aminé du N-acyl amino acide est choisi parmi la lysine, l'acide glutamique et l'alanine, de préférence l'acide aminé est l'acide glutamique ; et/ou le sel du N-acyl amino acide est choisi parmi les sels de sodium, de potassium, de lithium, de calcium, de magnésium, de strontium ; les sels d'aluminium, de zirconium, de zinc ; les sels d'ammonium et les sels d'un amino-alcool comme le sel de (2-hydroxy éthyl)ammonium.

11. Composition selon l'une des revendications précédentes, dans laquelle le N-acyl amino acide comprend un groupe acyle ayant de 8 à 22 atomes de carbone, tel qu'un groupe 2-éthylhexanoyle, caproyle, lauroyle, myristoyle, palmitoyle, stéaroyle ou cocoyle, de préférence le groupe acyle est un groupe stéaroyle.

12. Composition selon l'une des revendications précédentes, dans laquelle le N-acyl amino acide est un dérivé d'acide glutamique et/ou un de ses sels, et plus particulièrement un stéaroyl glutamate, de préférence le stéaroyl glutamate de disodium ou le stéaroyl glutamate d'aluminium.

13. Composition selon l'une des revendications précédentes, dans laquelle le pigment traité en surface est choisi parmi :
- le dioxyde de titane anatase enrobé de stéaroyl glutamate d'aluminium ;
- les oxydes de fer traités par le couple stéaroyl glutamate de disodium/hydroxyde d'aluminium; et
- les oxydes de fer jaune, rouge ou noir traités par le stéaroyl glutamate d'aluminium.

14. Composition selon l'une des revendications précédentes, dans laquelle le milieu aqueux physiologiquement acceptable comprend au moins 5% en poids d'eau par rapport au poids total de la composition, de préférence au moins 10% en poids, de préférence au moins 20% en poids, de préférence au moins 30% en poids, de préférence au moins 40% en poids ; de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50% ; et éventuellement au moins un solvant organique miscible dans l'eau à 25°C choisi parmi les alcools, les polyols et leurs mélanges.

15. Composition selon l'une des revendications précédentes, qui comprend au moins un alpha hydroxy acide, de préférence choisi parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

16. Composition selon l'une des revendications précédentes, qui présente un pH inférieur ou égal à 7, de préférence inférieur ou égal à 6,5, de préférence inférieur ou égal à 6,3, de préférence compris entre 3 et 6,3, de préférence compris entre 4 et 6,3.

17. Procédé de maquillage et/ou de soin de la peau et/ou des phanères, dans lequel on applique la composition selon l'une des revendications précédentes sur la peau et/ou les phanères.
